# EUROPEAN PATENT SPECIFICATION

(11) **EP 3 361 883 B1**
(45) Date of publication and mention of the grant of the patent: **24.07.2019**
(21) Application number: 16790260.0
(22) Date of filing: 14.10.2016
(51) Int. Cl.: A23L 5/00, A23P 10/00

(54) **CAPSULES FOR LOWERING SERUM CHOLESTEROL**
KAPSELN ZUR SENKUNG DES SERUMCHOLESTERINS
CAPSULES POUR RÉDUIRE LE CHOLESTÉROL SÉRIQUE

(30) Priority: 16.10.2015 EP 15002049
(43) Date of publication of application: 22.08.2018
(73) Proprietor: Raisio Nutrition Ltd, 21200 Raisio (FI)
(72) Inventor: KUUSISTO, Päivi, 21200 Raisio (FI); WESTER, Ingmar, 21200 Raisio (FI); LEE, Kyeong Jun, 21200 Raisio (FI)
(86) International application number: PCT/EP2016/001706
(87) International publication number: WO 2017/063746

(56) References cited:
- WO-A1-03/064444
- WO-A2-2007/094000
- WO-A2-2009/013395
- US-A1- 2003 129 253

## Description

### FIELD OF THE INVENTION

The present invention relates to the field of nutrition and health, and especially to capsules for lowering serum LDL cholesterol level.

### BACKGROUND OF THE INVENTION

Cardiovascular disease is counted among the most common diseases in Western countries and its occurrence is increasing also in Asian countries. The most important individual risk factor is elevated serum LDL cholesterol level, and therefore, lowering of the serum concentrations of LDL cholesterol is the most effective single measure regarding both prevention and effective treatment of cardiovascular disease.

The most important drugs for reduction of serum cholesterol levels are the statins, which function primarily by inhibiting the synthesis of cholesterol, mainly in the liver. The most common side effects of the statins are gastrointestinal. Other less common side effects include headache, dizziness, rash, and sleep disturbances. In addition, statins may cause both liver damage and muscle disorders, and they have been reported to increase the risk of type II diabetes.

As an alternative to cholesterol lowering drugs, or in addition to them, also life style changes can reduce the risk of cardiovascular diseases. In particular increasing physical exercise and/or adopting healthy diets recommended by governments or non-governmental associations are beneficial. An additional nutritional way to reduce serum LDL cholesterol levels is the use of cholesterol lowering functional foods that can be consumed as part of any conventional diet. This alternative has been greatly welcomed by consumers.

Food products enriched with components having a cholesterol lowering effect have been commercially available for almost 20 years. Such functional food products usually contain plant sterols and/or plant stanols and especially their fatty acid esters as active ingredients. Plant sterols have since the early 1950's been known to reduce serum cholesterol levels. US Patent 6 174 560 describes plant stanol fatty acid esters, a method for their preparation, and the cholesterol lowering effects thereof. An intake of 2 g per day of plant stanols is reported to lower serum LDL cholesterol levels in man up to 14%. Benecol® is a well-known trademark for plant stanol ester containing products and nowadays plant stanol ester is used in a variety of food products. Many of these products, e.g. margarine-type spreads and drinkable and spoonable yogurts, need continuous refrigeration. However, many consumers would prefer to have their daily dose of plant sterols and/or plant stanols available in a product that can be carried along wherever they go, e.g. as a dietary supplement. WO-A-2009/013395 discloses a method for preparing a beverage with improved stability. WO-A-03/064444 discloses fractionation of phytosterol esters in oil.WO-A-2007/094000 discloses applications of microencapsulated essential oils. US-A-2003/0129253 discloses aqueous suspensions of hydrophilic nutrients.

One of the most common types of dietary supplements are capsules, such as soft gelatin capsules, so called softgel capsules. Capsules consist of a shell and a filling containing the active ingredient.

Softgel capsules containing plant sterol ester and/or plant stanol ester have already been disclosed. The clinical trials conducted with these softgel capsules have yielded variable results. Most clinical studies with plant sterol ester and/or plant stanol ester softgel capsules have failed to deliver the expected serum LDL cholesterol lowering efficacy compared with the corresponding clinical trials with plant sterol ester and/or plant stanol ester functional food products. In a recent study of Ottestad et al. (Atherosclerosis 2013 June; 228(2):421-5) a softgel capsule containing plant sterol ester (2 g/d plant sterols) did not lower serum LDL cholesterol in hypercholesterolemic subjects. The authors concluded that this delivery system (softgel capsules) of plant sterol ester does not seem to give the expected LDL cholesterol reduction and thus the clinical relevance of the consumption of plant sterol ester capsules remains uncertain.

The softgel capsules that have been commercially available or are currently on the market contain plant sterol ester and/or plant stanol ester as such or mixed with vegetable oil, e.g. 80 % plant sterol ester and/or plant stanol ester and 20 % vegetable oil. Usually the recommended daily amount of plant sterols and/or plant stanols is 2 g, which means that the daily minimum required amount of plant sterol ester and/or plant stanol ester is about 3.4 g. Therefore the size of capsules is big, delivering about 1 g plant sterol ester and/or plant stanol ester in each soft gelatin capsule. Still several capsules are needed to provide the recommended daily amount of plant sterols and/or plant stanols. For optimal cholesterol lowering efficacy plant sterol ester and/or plant stanol ester capsules are recommended to be consumed with a meal. Because the clinical relevance of plant sterol ester and/or plant stanol ester capsules has been questioned, there remains a need for improved plant sterol ester and/or plant stanol ester capsules that will deliver the expected serum LDL cholesterol lowering effect when consumed as recommended.

### BRIEF DESCRIPTION OF THE INVENTION

The present invention is directed to a capsule comprising a shell and a filling, in which the filling comprises plant sterol ester and/or plant stanol ester in an amount of at least 80 % by weight and citrus oil in an amount of at least 2.0 % by weight.

The present invention is further directed to a method for preparing the capsule.

The present invention is still further directed to the capsule for use as a medicament or to the capsule for use in the treatment of high serum LDL cholesterol level and/or cardiovascular disease. The present invention is defined by the appended claims.

### DETAILED DESCRIPTION OF THE INVENTION

It is thus an object of the present invention to provide a capsule comprising a shell and a filling that contains plant sterol ester and/or plant stanol ester in an amount of at least 80 % by weight and citrus oil in an amount of at least 2.0 % by weight of the filling.

It has now been found that the disintegration of some prior known capsule fillings containing plant sterol ester and/or plant stanol ester in a simulated gastric fluid test at 38 °C is not optimal, but very slow and inefficient. Presumably the slow and inefficient disintegration has hampered the cholesterol lowering efficacy of these capsule products.

An efficient disintegration of the plant sterol ester and/or plant stanol ester capsule filling in the gastric environment is naturally a prerequisite for the serum LDL cholesterol lowering effect. The main mechanism of action of plant sterols and plant stanols is suggested to be through competitively inhibiting cholesterol incorporation in the mixed micelles of bile salts. The gall bladder contraction and subsequent bile secretion, as well as simultaneous delivery of plant stanol ester or plant sterol ester and pancreatic enzymes including cholesterol esterase into the upper part of the duodenum is necessary for the cholesterol lowering efficacy of plant sterols and plant stanols. Thus the efficiency and timing of the disintegration of the plant sterol ester and/or plant stanol ester capsule filling in the stomach is important for the LDL cholesterol lowering effect of the plant sterol ester and/or plant stanol ester filling.

It is also advantageous to have a filling with plant sterol ester and/or plant stanol ester content of more than 80 % by weight of the filling, as the recommended daily amount of plant sterols and/or plant stanols can then be obtained from a smaller number of capsules and/or from smaller size capsules.

Thus the present invention provides a capsule comprising a shell and a filling containing a high amount of plant sterol ester and/or plant stanol ester, in which capsule the filling is disintegrated fast in the stomach or in conditions resembling the gastric conditions. It has now surprisingly been found that a capsule filling containing plant sterol ester and/or plant stanol ester and citrus oil in certain amounts is disintegrated fast in a simulated gastric fluid test. It is now hypothesized that the fast disintegration of the capsule filling of the invention contributes to a good serum LDL cholesterol lowering efficacy of capsules containing this filling.

An advantage of the capsule filling of the present invention is that it contains a high concentration of plant sterol ester and/or plant stanol ester. Thus the consumer needs to take only a few capsules per day to obtain the daily effective dose of plant sterol ester and/or plant stanol ester. All the ingredients used in the capsule filling of the present invention are food grade.

Thus, the present invention is preferably directed to a capsule consisting of a shell and a filling comprising plant sterol ester and/or plant stanol ester in an amount of at least 80 % by weight and citrus oil in an amount of at least 2.0 % by weight of the filling.

By "capsule" is in this disclosure meant dosage forms which consist of hard or soft shells of various shapes and filling volumes, and a filling containing the active ingredient. Capsules are intended for oral administration. Hard capsules have shells consisting of two cylindrical sections, one end of which is rounded and closed, the other open. The filling is put into one of the sections which is then closed by slipping the other section over it. If the filling is liquid or semi-solid, the capsule halves are sealed together. The hard shell is usually made of gelatin and water together with colouring and opacifying agent. The present invention is especially directed to soft capsules, which are also called "softgel capsules". By "softgel capsules" is meant one-piece, hermetically sealed capsules. The softgel capsule shell is usually based on gelatin, water and plasticiser(s), and optionally also colouring and opacifying agent. For example glycerol and/or sorbitol or its derivatives can be used as a plasticiser. There are also polysaccharide-based softgel capsule shells that contain e.g. starch and/or carrageenan. The softgel capsules can be manufactured by the so called rotary-die encapsulation process. In this process, the encapsulation of the filling is made simultaneously with the formation of the capsule shell. First two plasticized films called ribbons are formed from the shell mass. Each ribbon is passed over a die and sealed to the other ribbon at the point where the two dies meet while filled simultaneously. Then the filled capsules are dried.

By "filling", i.e. "capsule filling", is meant the fill composition inside the capsule shell. In this disclosure, the filling contains the active ingredient, i.e. plant sterol ester and/or plant stanol ester.

By "disintegration" is in this disclosure meant disintegration of the capsule filling in a simulated gastric fluid test. In the test a simulated gastric fluid is used as the liquid medium. It has low pH (e.g. 1-2). The speed of disintegration of the capsule filling can be monitored as e.g. described in the examples of this disclosure. The capsule filling is first produced and let to crystallize at room temperature for at least 70 hours in moulds as described in the Examples of this disclosure. The capsule filling is then placed in the simulated gastric fluid at 38 °C with mixing (e.g. 150-160 oscillations per minute), and its disintegration is visually observed. The time when the capsule filling is disintegrated (i.e. it is broken into several disintegrated particles and/or forms a cloudy liquid) is used as a measure of the speed of disintegration. As used here, the definition of disintegration does not take into account the disintegration of the capsule shell, but only the capsule filling. However, for optimal cholesterol lowering effect also the capsule shell shall disintegrate as fast as possible. Determination of disintegration times of the capsule shell can be carried out according to US Pharmacopeia USP29-NF24. The shell is preferably disintegrated within 15 minutes. Such shells are known to a skilled person in the art.

When used here in connection with the disintegration test, by "disintegrated particle" is meant visually distinguishable pieces and/or droplets of the filling that have disintegrated from the filling in the simulated gastric fluid test. The disintegrated particle can be in solid, semi-solid or liquid form.

The capsule filling of the present invention disintegrates fast in the stomach in order to provide an effective serum cholesterol lowering in humans. Preferably several disintegrated particles and/or a cloudy liquid is formed of the capsule filling in the simulated gastric fluid test within 20 minutes, more preferably within 15 minutes, and most preferably within 10 minutes.

The capsule filling of the present invention contains at least 80 % %, preferably at least 82 %, more preferably at least 85 %, still more preferably at least 87 % and most preferably at least 90 % by weight plant sterol ester and/or plant stanol ester. The capsule filling contains preferably at most 98 %, more preferably at most 97 %, still more preferably at most 96 %, even more preferably at most 95 % and most preferably at most 94 % by weight plant sterol ester and/or plant stanol ester.

As used here, the term "plant sterol ester and/or plant stanol ester" refers to plant sterols and/or plant stanols in esterified form. The term "plant sterol" includes 4-desmethyl sterols and 4-monomethyl sterols and the term "plant stanol" includes 4-desmethyl stanols and 4-monomethyl stanols. Typical 4-desmethyl sterols are sitosterol, campesterol, stigmasterol, brassicasterol, 22-dehydro-brassicasterol and δ5-avenasterol. Typical stanols are sitostanol, campestanol and their C24-epimers. The term "plant sterols and/or plant stanols" includes all possible mixtures of named sterols and/or stanols as well as any individual sterol and/or stanol.

In this invention plant sterols and/or plant stanols are esterified with a carboxylic acid or with a blend of carboxylic acids and are called "plant sterol ester and/or plant stanol ester". Examples of suitable carboxylic acids are fatty acids. The fatty acids are aliphatic, have 4-24 carbon atoms, and are saturated, monounsaturated or polyunsaturated. The physical properties of the plant sterol ester and/or plant stanol ester can be modified by changing the fatty acid moiety of the molecule. Preferably the plant sterols and/or plant stanols are esterified with vegetable oil based fatty acids. Preferred are fatty acids of rapeseed oil, soybean oil, sunflower oil and corn oil. Preferably plant sterol ester and/or plant stanol ester has a solid fat content (SFC) of at least 20 %, more preferably at least 25 %, still more preferably at least 30 %, and most preferably at least 35 % at 20 °C. The solid fat content can be measured e.g. by conventional NMR technique by using a serial tempering method starting at 10 °C.

Most preferred are plant stanol fatty acid esters. Therefore, the plant sterol ester and/or plant stanol ester contains preferably plant stanol ester in an amount of at least 30 %, more preferably at least 50 %, still more preferably at least 70 % and most preferably at least 90 % by weight. Preferably the plant sterol ester and/or plant stanol ester has a solid fat content (SFC) of at least 20 %, more preferably at least 25 %, still more preferably at least 30 %, and most preferably at least 35 % at 20 °C and contains plant stanol ester in an amount of at least 30 %, more preferably at least 50 %, still more preferably at least 70 % and most preferably at least 90 % by weight.

Plant stanol fatty acid ester and the cholesterol lowering effects thereof, as well as a suitable method for its preparation, are disclosed in e.g. US Patent 6,174,560. Obviously plant sterol esters can also efficiently be produced by the production method disclosed in US 6 174 560. Alternatively fatty acid esters of plant sterols and/or plant stanols can be produced by any suitable food grade method disclosed in the art. Commercially available plant sterol ester and/or plant stanol ester ingredients e.g. from Raisio Nutrition or BASF can be used.

The content of the plant sterol ester and/or plant stanol ester in the capsule filling can be analyzed by e.g. the method described by Lubinus et al. (Eur. J. Nutr. 2013, 52(3):997-1013) or by Esche et al. (J. Agric. Food Chem. 2012, 30; 60(21):5330-9).

The capsule filling of the present invention contains at least 2.0 % citrus oil. As used here the term "citrus oil" refers to food grade or pharmaceutical grade essential oil obtained from citrus fruits belonging to Rutaceae family. Conventionally food grade essential oils from citrus fruits have been used as flavoring agents. The essential oil is obtained from the pericarp, peel, pulp or juice of citrus fruits. In some cases also flowers, twigs or leaves can be used as a raw material. Preferably the citrus oil is obtained from the peel. Citrus oil can be obtained for example by mechanical processes, distillation or extraction techniques. Mechanical processes such as cold pressing are preferred. Main components in citrus oil are terpenes, especially monoterpene D-limonene. As used here, the term "citrus oil" includes also terpene fractions that are purified from the essential oils of citrus fruits. This kind of fractions may contain for example at least 93 %, and preferably at least 95 % by weight D-limonene.

Preferably the citrus oil of this invention is selected from orange oil, grapefruit oil, mandarin oil, lemon oil, lime oil, pomelo oil, bitter orange oil or mixtures thereof. More preferably the citrus oil is selected from orange oil (also called sweet orange oil), grapefruit oil, mandarin oil or mixtures thereof. Still more preferably the citrus oil is orange oil (also called sweet orange oil). Preferably the citrus oil of the invention contains at least 70 %, more preferably at least 80 % and most preferably at least 90 % by weight D-limonene. Most preferably the citrus oil is orange oil that contains at least 90 % by weight D-limonene.

It was found that capsule fillings having a high concentration of plant sterol ester and/or plant stanol ester, and still fast disintegration in simulated gastric conditions, can be prepared by using citrus oil in certain concentrations. Thus the capsule filling according to the invention contains at least 2.0 %, preferably at least 3.0 %, more preferably at least 4.0 %, still more preferably at least 5.0 % and most preferably at least 6.0 % by weight citrus oil. Preferably the capsule filling contains at most 20 %, more preferably at most 18 %, still more preferably at most 15 %, even more preferably at most 13 % and most preferably at most 10 % by weight citrus oil.

Especially suitable capsule fillings can be prepared by using a certain weight ratio of plant sterol ester and/or plant stanol ester to citrus oil. Preferably in the capsule filling the weight ratio of plant sterol ester and/or plant stanol ester to citrus oil is at least 5:1, more preferably at least 7:1, still more preferably at least 9:1 and most preferably at least 11:1. Preferably in the capsule filling the weight ratio of plant sterol ester and/or plant stanol ester to citrus oil is at most 45:1, more preferably at most 30:1, still more preferably at most 25:1 and most preferably at most 18:1.

In a preferred embodiment, the capsule filling comprises 82-97 % by weight plant sterol ester and/or plant stanol ester and 3.0-18 % by weight citrus oil. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 5:1, more preferably at least 7:1, and most preferably at least 9:1. The weight ratio of plant sterol ester and/or plant stanol ester to citrus oil is preferably at most 30:1, more preferably at most 25:1. Preferably the capsule filling of this embodiment is a softgel capsule filling.

In another preferred embodiment, the capsule filling comprises 87-95 % by weight plant sterol ester and/or plant stanol ester and 5.0-13 % by weight citrus oil. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 7:1, and more preferably at least 9:1. The weight ratio of plant sterol ester and/or plant stanol ester to citrus oil is preferably at most 18:1.

Optionally the capsule filling of the present invention may further contain an emulsifier. As used here the term "emulsifier" refers to a substance classified as an emulsifier in food legislation (e.g. Regulation (EC) No 1333/2008) or a mixture of such substances. The characterizing feature of the emulsifier is a structure in which one portion of the molecule is polar (hydrophilic) and the other non-polar (hydrophobic). As used here, the emulsifier is a food grade substance, and its usage in food supplements is allowed by regulations (e.g. Regulation (EC) No 1333/2008). The capsule filling according to the invention may preferably contain 0-15 %, more preferably 0.1-15 %, still more preferably 1.0-13 %, even more preferably 2.0-10 %, and most preferably 3.0-8.0 % by weight emulsifier. If the capsule filling contains an emulsifier, the weight ratio of plant sterol ester and/or plant stanol ester to emulsifier is preferably at least 6:1, more preferably at least 7:1, still more preferably at least 9:1, and most preferably at least 11:1.

One type of emulsifier or a mixture of at least two emulsifiers can be used. Typical examples of suitable emulsifiers include monoglycerides, such as distilled monoglycerides; diglycerides; monoglyceride esters such as acetic, lactic, succinic, citric or diacetyl tartaric acid esters of monoglycerides; lecithins; modified lecithins such as lysolecithins; polyglycerol esters; polysorbates; sorbitan esters; propylene glycol esters; sugar esters; and mixtures of any thereof. Preferably the emulsifier is selected from the group consisting of monoglycerides; diglycerides; lactic, citric or diacetyl tartaric acid esters of monoglycerides; lecithins; modified lecithins; polyglycerol esters, polysorbates, sugar esters and mixtures of any thereof.

Suitably the emulsifier contains at least 30 %, preferably at least 50 %, more preferably at least 70 % by weight monoglycerides and most preferably the emulsifier consists essentially of monoglycerides (i.e. at least 95 % by weight). The emulsifier may also be a mixture of monoglycerides and diglycerides, wherein the amount of monoglycerides is at least 30 %, preferably at least 50 %, more preferably at least 70 % and most preferably at least 95 % by weight of the total amount of emulsifier. Preferably the emulsifier contains at least 30 %, preferably at least 50 %, more preferably at least 70 % and most preferably at least 95 % monoglycerides having a melting temperature of at most 70 °C, preferably at most 65 °C, most preferably at most 60 °C and an iodine value of at least 30, more preferably at least 40, and most preferably at least 50. By melting temperature is meant the temperature at which the monoglyceride is completely melted.

In a preferred embodiment, the capsule filling comprises 82-96 % by weight plant sterol ester and/or plant stanol ester, 3.0-15 % by weight citrus oil, and 0.1-15 % by weight emulsifier. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 5:1, more preferably at least 7:1 and most preferably at least 9:1. The weight ratio of plant sterol ester and/or plant stanol ester to citrus oil is preferably at most 30:1 and more preferably at most 25:1. The weight ratio of plant sterol ester and/or plant stanol ester to emulsifier is preferably at least 6:1, more preferably at least 7:1 and most preferably at least 9:1. Preferably the capsule filling of this embodiment is a softgel capsule filling.

In another preferred embodiment, the capsule filling comprises 87-94 % by weight plant sterol ester and/or plant stanol ester, 5.0-10 % by weight citrus oil, and 1.0-8.0 % by weight emulsifier. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 9:1. The weight ratio of plant sterol ester and/or plant stanol ester to citrus oil is preferably at most 18:1. The weight ratio of stanol ester to emulsifier is preferably at least 11:1. Preferably the capsule filling of this embodiment is a softgel capsule filling.

Optionally the capsule filling according to the present invention may further contain free plant sterols and/or free plant stanols in preferably an amount of 0-10 %, more preferably 0.05-10 %, still more preferably 0.05-5.0 %, and most preferably 0.05-3.0 % by weight of the filling. Preferably the capsule filling contains at least 0.5 %, more preferably at least 1.0 % and most preferably at least 2.0 % by weight free plant sterols and/or free plant stanols. By "free plant sterols and/or free plant stanols" is meant plant sterols and/or plant stanols in free form i.e. as an alcohol. This means that the hydroxyl group at carbon atom number 3 is free, not forming an ester or glycoside bond. The term "plant sterols and/or plant stanols" includes the same individual plant sterols and/or plant stanols and their mixtures as defined earlier in this invention. The free plant sterols and/or free plant stanols contained in many commercial plant sterol ester products and/or plant stanol ester products may during a prolonged storage (e.g. 6 months) of the gelatin capsule, form beneath the gelatin shell a hard free plant sterol and/or free plant stanol layer, which slows down the disintegration. However, according to the present invention, it is possible to include free plant sterols and/or free plant stanols in the capsule filling without hampering its fast disintegration.

In a preferred embodiment, the capsule filling comprises 82-96 % by weight plant sterol ester and/or plant stanol ester, 3.0-15 % by weight citrus oil, 0.1-15 by weight % emulsifier, and 0.05-5.0 % by weight free plant sterols and/or free plant stanols. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 5:1, more preferably at least 7:1 and most preferably at least 9:1. The weight ratio of plant sterol ester and/or plant stanol ester to citrus oil is preferably at most 30:1 and more preferably at most 25:1. The weight ratio of plant sterol ester and/or plant stanol ester to emulsifier is preferably at least 6:1, more preferably at least 7:1 and most preferably at least 9:1. Preferably the capsule filling of this embodiment is a softgel capsule filling.

In another preferred embodiment, the capsule filling comprises 87-94 % by weight plant sterol ester and/or plant stanol ester, 5.0-10 % by weight citrus oil, 1.0-8.0 % by weight emulsifier, and 0.05-3.0 % by weight free plant sterols and/or free plant stanols. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 9:1. The weight ratio of plant sterol ester and/or plant stanol ester to citrus oil is preferably at most 18:1. The weight ratio of stanol ester to emulsifier is preferably at least 11:1. Preferably the capsule filling of this embodiment is a softgel capsule filling.

The capsule filling of the present invention may further contain edible fat. By "edible fat" is here meant edible fats and oils that consist mainly (at least 90 %, preferably at least 95 % and most preferably at least 98 % by weight) of triacylglycerols and that are suitable for human consumption. The edible fat can for example be commercially available vegetable oil, vegetable fat, marine oil (e.g. fish oil) or algae oil. It can be naturally occurring, i.e. un-modified but refined, bleached and deodorised, or modified, e.g. hydrogenated, fractionated, transesterified or contain structured triacylglycerols. Edible fat can also be a mixture of different edible fats. Preferably the edible fat is a vegetable oil, a blend of vegetable oils or a fish oil. Examples of suitable vegetable oils are canola/rapeseed oil, soybean oil, sunflower oil, olive oil, and corn oil. The citrus oil used in the present invention is obviously not included in the term edible fat due to its high content of limonene and thus low content of triacylglycerols.

Especially suitable capsule fillings can be prepared when the content of the edible fat is kept low or especially when essentially no edible fat is present in the filling. Preferably the capsule filling of the present invention may contain 0-8.0 % by weight edible fat. More preferably the capsule filling contains 0.1-8.0 %, still more preferably 0.1-4.0 %, even more preferably 0.1-2.0 % by weight edible fat. Most preferably the capsule filling of the present invention contains essentially no edible fat (i.e. 0 to less than 0.1 % by weight edible fat). If the capsule filling contains edible fat, the weight ratio of plant sterol ester and/or plant stanol ester to the edible fat is preferably at least 11:1. More preferably the weight ratio of plant sterol ester and/or plant stanol ester to the edible fat is at least 22:1, still more preferably at least 33:1, most preferably at least 44:1. If the capsule filling contains edible fat, the weight ratio of citrus oil to the edible fat is preferably at least 0.3:1, more preferably at least 0.5:1, still more preferably at least 1.0:1, even more preferably at least 1.5:1, and most preferably at least 2.5:1.

If the capsule filling contains both emulsifier and edible fat, the weight ratio of plant sterol ester and/or plant stanol ester to the summed amount of emulsifier and edible fat is preferably at least 6:1, more preferably at least 7:1, still more preferably at least 9:1 and most preferably at least 11:1.

In a preferred embodiment, the capsule filling comprises 82-96 % by weight plant sterol ester and/or plant stanol ester, 3.0-15 % by weight citrus oil, 0.05-5.0 % free plant sterols and/or free plant stanols, 0.1-15 % by weight emulsifier, and 0.1-8.0 % by weight edible fat. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 5:1, more preferably at least 7:1 and most preferably at least 9:1. The weight ratio of plant sterol ester and/or plant stanol ester to citrus oil is preferably at most 30:1 and more preferably at most 25:1. The weight ratio of plant sterol ester and/or plant stanol ester to emulsifier is preferably at least 6:1, more preferably at least 7:1 and most preferably at least 9:1. The weight ratio of plant sterol ester and/or plant stanol ester to the edible fat is preferably at least 11:1, and most preferably at least 22:1. The weight ratio of citrus oil to the edible fat is preferably at least 0.5:1, and most preferably at least 1:1. The weight ratio of plant sterol ester and/or plant stanol ester to the summed amount of emulsifier and edible fat is preferably at least 6:1, more preferably at least 7:1, still more preferably at least 9:1 and most preferably at least 11:1. Preferably the capsule filling of this embodiment is a softgel capsule filling.

In another preferred embodiment, the capsule filling comprises 87-94 % by weight plant sterol ester and/or plant stanol ester, 5.0-10 % by weight citrus oil, 0.05-3.0 % free plant sterols and/or free plant stanols, 1.0-8.0 % by weight emulsifier, and 0.1-2.0 % by weight edible fat. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 9:1. The weight ratio of plant sterol ester and/or plant stanol ester to citrus oil is preferably at most 18:1. The weight ratio of stanol ester to emulsifier is preferably at least 11:1. The weight ratio of plant sterol ester and/or plant stanol ester to the edible fat is preferably at least 44:1. The weight ratio of citrus oil to the edible fat is preferably at least 2.5:1. The weight ratio of plant sterol ester and/or plant stanol ester to the summed amount of emulsifier and edible fat is preferably at least 9:1 and most preferably at least 11:1. Preferably the capsule filling of this embodiment is a softgel capsule filling.

In still another preferred embodiment, the capsule filling comprises 85-94 % by weight plant sterol ester and/or plant stanol ester, at least 3.0-13 % by weight citrus oil, 0.05-5.0 % free plant sterols and/or free plant stanols, and 0.1-8.0 % by weight emulsifier. The capsule filling of this embodiment contains essentially no edible fat (i.e. 0 to less than 0.1 % by weight edible fat). Preferably the capsule filling of this embodiment comprises at least 4.0 %, more preferably at least 5.0 % and most preferably at least 6.0 % by weight citrus oil. Preferably the capsule filling comprises at most 10 % by weight citrus oil. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 7:1, most preferably at least 9:1. The weight ratio of plant sterol ester and/or plant stanol ester to citrus oil is preferably at most 30:1 and more preferably at most 25:1. The weight ratio of stanol ester to emulsifier is preferably at least 11:1. The weight ratio of plant sterol ester and/or plant stanol ester to the summed amount of emulsifier and edible fat is preferably at least 11:1. Preferably the capsule filling of this embodiment is a softgel capsule filling.

In still another preferred embodiment, the capsule filling comprises 87-94 % by weight plant sterol ester and/or plant stanol ester, at least 3.0-13 % by weight citrus oil, 0.05-5.0 % free plant sterols and/or free plant stanols, and 0.1-8.0 % by weight emulsifier. The capsule filling of this embodiment contains essentially no edible fat (i.e. 0 to less than 0.1 % by weight edible fat). Preferably the capsule filling of this embodiment comprises at least 4.0 %, and most preferably at least 5.0 % by weight citrus oil. Preferably the capsule filling comprises at most 10 % by weight citrus oil. Preferably the citrus oil is orange oil, grapefruit oil, mandarin oil, lemon oil, lime oil, pomelo oil, bitter orange oil or mixtures thereof. More preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. The weight ratio of plant sterol ester and/or plant stanol ester to citrus oil is preferably at least 7:1, most preferably at least 9:1. The weight ratio of plant sterol ester and/or plant stanol ester to citrus oil is preferably at most 30:1 and more preferably at most 25:1. The weight ratio of stanol ester to emulsifier is preferably at least 11:1. The weight ratio of plant sterol ester and/or plant stanol ester to the summed amount of emulsifier and edible fat is preferably at least 11:1. Preferably the capsule filling of this embodiment is a softgel capsule filling.

The capsule filling of the invention may also contain optional other ingredients, e.g. fat-soluble ingredients with beneficial health effects, such as fat soluble vitamins (e.g. vitamin D), tocopherols, tocotrienols, coenzyme Q10, K vitamins, antioxidants and mixtures of any thereof.

Preferably the capsule filling of the present invention does not contain water (i.e. it may contain at most 0.1 % by weight water). The capsule filling is preferably free of excipients. These are often used in pharmaceutical capsules and tablets. Examples of such excipients include silicon dioxide, microcrystalline cellulose, starch, maltodextrin and sugars. The emulsifier, the optional edible fat, as well as the optional free plant sterols and/or free plant stanols, all defined in the present invention, are obviously hereby excluded from the term excipient.

Another object of the present invention is to provide a method of preparing the capsule according to the present invention. The method comprises at least the following steps:
a) heating plant sterol ester and/or plant stanol ester, and optionally emulsifier, free plant sterols and/or free plant stanols, edible fat and/or optional other ingredients, preferably to a temperature of at least 40 °C, more preferably to 40-80 °C, to obtain a clear solution, and keeping the solution at this temperature for at least 10 minutes,
b) mixing citrus oil and plant sterol ester and/or plant stanol ester, and optionally emulsifier, free plant sterols and/or free plant stanols, edible fat and/or optional other ingredients,
c) keeping the temperature of the mixture such that the mixture remains clear, and using the clear mixture to fill a capsule, preferably a softgel capsule.

In the method plant sterol ester and/or plant stanol ester is heated until it is totally melted and a clear solution is obtained. Often the commercial plant sterol ester and/or plant stanol ester ingredient contains both plant sterol ester and/or plant stanol ester and free plant sterols and/or free plant stanols. If free plant sterols and/or free plant stanols are used, they can be suspended in the plant sterol ester and/or plant stanol ester. The melt is kept at 40-80 °C for at least about 10 minutes to destroy the so called crystal memory of the plant sterol ester and/or plant stanol ester. If emulsifier is used, it is also melted, if it is not liquid at room temperature. If edible fat is used, it is also melted, if it is not liquid at room temperature. All ingredients (including optional other ingredients) are then mixed. It is also possible to first mix plant sterol ester and/or plant stanol ester with one or more optional ingredient selected from emulsifier, free plant sterols and/or free plant stanols, edible fat and optional other ingredients, and then perform the melting step a). It is further possible to first melt plant sterol ester and/or plant stanol ester and one or more optional ingredient selected from emulsifier, free plant sterols and/or free plant stanols, edible fat and other optional ingredients, add the citrus oil and continue the melting process until the mixture is totally melted. Thus the step b) may precede step a) or be performed simultaneously with step a). Important is however that the plant sterol ester and/or plant stanol ester is in melted form long enough to destroy its crystal memory. After mixing all the ingredients together the mixture is still kept in melted form preferably with gentle stirring for at least about 10 minutes to ensure the mixture is homogenous. The mixture is then dosed into a capsule manufacturing line preferably at a temperature of about at least 10 °C above the temperature at which this mixture becomes clear (i.e. totally melted). Finally, capsules are preferably formed by producing a shell around the dosed mixture at the encapsulation line.

Because the capsule filling of the invention has a high plant sterol ester and/or plant stanol ester content, the daily effective dose of plant sterol ester and/or plant stanol ester can be incorporated in a few capsules. The preferred daily dose of plant sterol ester and/or plant stanol ester is at least 0.8 g, more preferably at least 1.3 g, still more preferably at least 1.7 g, even more preferably at least 2.5 g and most preferably the daily dose of plant sterol ester and/or plant stanol ester is at least 3.4 g. The preferred daily dose of plant sterol ester and/or plant stanol ester is at most 10 g, more preferably at most 8 g, still more preferably at most 7 g, even more preferably at most 6 g, and most preferably at most 5 g.

The daily dose of plant sterol ester and/or plant stanol ester is preferably provided by 0.8-13 g of the capsule filling, i.e. the daily dose of the capsule filling is preferably 0.8-13 g. More preferably the daily dose of plant sterol ester and/or plant stanol ester is provided by 1.4-10 g, still more preferably 1.8-9 g, even more preferably 2.6-8 g, and most preferably 3.6-6 g of the softgel capsule filling. Preferably the daily dose of plant sterol ester and/or plant stanol ester is provided by 1-15 capsules, i.e. the recommended daily number of the capsules is 1-15. More preferably the daily dose of plant sterol ester and/or plant stanol ester is provided by 1-12, still more preferably by 1-10, even more preferably by 1-7, and most preferably by 1-4 capsules. Softgel capsules can have any shape. The most commons softgel capsule shapes are e.g. round, oval and oblong, but also special shapes, such as animal shapes, can be produced.

In a preferred embodiment, the capsule filling comprises 85-94 % by weight plant sterol ester and/or plant stanol ester, 3.0-13 % by weight citrus oil, 0.05-5.0 % by weight free plant sterols and/or free plant stanols, and 0.1-8.0 % by weight edible fat. The capsule filling of this embodiment contains essentially no emulsifier (i.e. 0 to less than 0.1 % by weight emulsifier). Preferably the capsule filling of this embodiment comprises at least 4.0 %, more preferably at least 5.0 %, and most preferably at least 6.0 % by weight citrus oil. Preferably the capsule filling comprises at most 10 % by weight citrus oil. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. In this embodiment, the weight ratio of plant sterol ester and/or plant stanol ester to the citrus oil is preferably at least 7:1 and most preferably at least 9:1. The weight ratio of plant sterol ester and/or plant stanol ester to citrus oil is preferably at most 30:1 and most preferably at most 25:1. The edible fat of this embodiment is preferably vegetable oil or fish oil. The weight ratio of the plant sterol ester and/or plant stanol ester to the edible fat is preferably at least 11:1. The weight ratio of citrus oil to the edible fat is preferably at least 0.5:1. Preferably the capsule filling of this embodiment is a softgel capsule filling.

In another preferred embodiment, the capsule filling comprises 87-94 % by weight plant sterol ester and/or plant stanol ester, 3.0-13 % by weight citrus oil, 0.05-5.0 % by weight free plant sterols and/or free plant stanols, and 0.1-8.0 % by weight edible fat. The capsule filling of this embodiment contains essentially no emulsifier (i.e. 0 to less than 0.1 % by weight emulsifier). Preferably the capsule filling of this embodiment comprises at least 4.0 %, and most preferably at least 5.0 % by weight citrus oil. Preferably the capsule filling comprises at most 10 % by weight citrus oil. Preferably the citrus oil is orange oil, grapefruit oil, mandarin oil or mixtures thereof. Most preferably the citrus oil is orange oil. Preferably the citrus oil contains at least 90 % by weight D-limonene. Preferably in this embodiment, plant sterol ester and/or plant stanol ester has a solid fat content (SFC) of at least 20 %, more preferably at least 25 %, still more preferably at least 30 %, and most preferably at least 35 % at 20 °C. Preferably plant sterol ester and/or plant stanol ester contains plant stanol ester in an amount of at least 30 %, more preferably at least 50 %, still more preferably at least 70 % and most preferably at least 90 % by weight of the plant sterol ester and/or plant stanol ester. In this embodiment, the weight ratio of plant sterol ester and/or plant stanol ester to the citrus oil is preferably at least 7:1 and most preferably at least 9:1. The weight ratio of plant sterol ester and/or plant stanol ester to citrus oil is preferably at most 30:1 and most preferably at most 25:1. The edible fat of this embodiment is preferably vegetable oil or fish oil. The weight ratio of the plant sterol ester and/or plant stanol ester to the edible fat is preferably at least 11:1. The weight ratio of citrus oil to the edible fat is preferably at least 0.5:1. Preferably the capsule filling of this embodiment is a softgel capsule filling.

A further object of the present invention is the before mentioned capsule for use as a medicament, especially for lowering serum LDL cholesterol. In other words, the invention relates to a capsule according to the present invention for use as a medicament or for use in the treatment of high serum LDL cholesterol level and/or for use in the treatment of cardiovascular disease. By medicament is here meant a substance that is physiologically active in preventing or alleviating a disease or a risk factor of a disease (a prophylactic use). The invention is further directed to a method for lowering serum LDL cholesterol in a subject in need thereof, wherein the capsule according to the invention is administered to the subject. Thereby the subject ingests capsules according to the invention in such an amount that an effective amount of plant sterol ester and/or plant stanol ester is ingested, as disclosed in this description.

In this description the amounts given in percentages mean percentage by weight (wt-%) of the capsule filling unless otherwise stated. Both comprising and containing mean "containing at least" in this context. Consisting of is a closed definition and means "is".

The invention will be described in greater detail by means of the following non-limiting examples.

### EXAMPLE 1

Two capsule fillings were prepared. The filling R1 was a reference capsule filling containing plant stanol ester and vegetable oil. The filling T1 was a filling according to the invention containing plant stanol ester and citrus oil. The disintegration of these fillings was tested in simulated gastric fluid.

Commercial plant stanol ester ingredient was used. It contained 0.4 % free stanols and 99.6 % plant stanol ester. Plant stanol ester was esterified with rapeseed oil fatty acids. The plant stanol ester ingredient was melted at 60 °C and kept at this temperature for 15 minutes after complete melting. Rapeseed oil and orange oil were heated to 40 °C. Rapeseed oil or orange oil was mixed with the melted plant stanol ester ingredient. The mixtures were then dosed into moulds of the size that provided a solidified filling of 1 g. Both R1 and T1 were allowed to cool and solidify for 70 hours at room temperature (22 °C).

| | R1 comparative | T1 |
|---|---|---|
| INGREDIENTS | wt % of the filling | |
| Plant stanol ester ingredient* | 90 | 90 |
| Rapeseed oil | 10 | |
| Citrus oil (Cold-pressed orange oil, contains >90 % D-limonene) | | 10 |
| Plant stanol ester to citrus oil -ratio | | 9:1 |

| | | |
|---|---|---|
| *containing 99.6 % by weight plant stanol ester, i.e. the plant stanol ester concentration in recipes R1 and T1 was 89.6 % by weight | | |

Simulated gastric fluid, pH 1.2, was prepared according to the instructions of United States Pharmacopeia for Simulated Gastric Fluid TS. Sodium chloride (2 g) was dissolved in hydrochloric acid (7 ml) and water (987.8 ml). The fluid was tempered at 38° C in a shaking water bath and pepsin (3.2 g purified pepsin 2000 FIR-U/g, EC 3.4.23.1 Merck) was added. The fluid was let to clarify for two minutes in the water bath. 300 ml of the fluid was transferred to a tempered bottle. 1 g of the filling (R1 or T1) was expelled from the mould and added into the simulated gastric fluid in the bottle and the bottle was placed in a shaking water bath (38° C, shaking 155 oscillations per minute). The disintegration of the filling was visually continuously observed for a period of 20 minutes.

| | R1 comparative | T1 |
|---|---|---|
| DISINTEGRATION TEST (time in minutes) | | |
| Stage 1: The filling is as one particle and there is no change in its appearance | From 0 min to the time of Stage 2 | From 0 min to the time of Stage 2 |
| Stage 2: Appearance of the filling has turned transparent, but the filling is still as one particle | 7 min | 1 min |
| Stage 3: Several (>2) disintegrated particles in the fluid | remained as one particle | 2 min |
| Stage 4: The fluid is cloudy | no | 6 min |

| | | |
|---|---|---|
| no = this stage was not reached during the follow-up time | | |

The reference capsule R1 filling was not disintegrated during the 20 min follow-up period in the simulated human gastric fluid test. After the 20 min continuous follow-up period, the R1 filling was left in the simulated human gastric fluid at 38 °C and observed every half hour until 3 hours had passed from the beginning of the test. The filling did not disintegrate (reach stage 3 or 4 in the disintegration test) even during this 3 hour period. Thus the disintegration of the R1 filling, representing the prior known plant sterol ester and/or plant stanol ester capsule fillings, was very slow and inefficient in the simulated gastric fluid. This slow disintegration in the simulated gastric fluid test indicates that this filling is not effectively disintegrated in the stomach. This may partly explain the poor or lower than expected serum LDL cholesterol lowering results obtained in published clinical trials with plant sterol ester and/or plant stanol ester softgel capsules.

The T1 filling according to the invention was disintegrated in the simulated gastric fluid very fast. Disintegrated particles could be visually distinguished already in 2 minutes. The simulated gastric fluid turned cloudy in 6 minutes, indicating that the filling was disintegrated into fine particles that were distributed throughout the fluid. Surprisingly the disintegration of the test filling was much faster than the disintegration of the corresponding reference filling R1. Both fillings R1 and T1 were solid at room temperature before the disintegration test. With the test filling T1 thus both a high plant stanol ester content and a fast disintegration can be achieved.

### EXAMPLE 2

Four capsule fillings (T2-T5) were prepared of plant stanol ester and citrus oil. The plant stanol ester and citrus oil were the same as used in Example 1. The capsule fillings were prepared as described in Example 1. All fillings were solid at room temperature before the disintegration test.

| | T2 | T3 | T4 | T5 |
|---|---|---|---|---|
| INGREDIENTS | wt % of the filling | | | |
| Plant stanol ester ingredient* | 98 | 96 | 95 | 87 |
| Citrus oil (Cold-pressed orange oil, contains >90 % D-limonene) | 2 | 4 | 5 | 13 |
| Plant stanol ester to citrus oil -ratio | 49:1 | 24:1 | 19:1 | 6.7:1 |

| | | | | |
|---|---|---|---|---|
| *containing % by weight plant stanol ester, i.e. the plant stanol ester concentration in recipes T2-T5 was 97.6 %, 95.5 %, 94.6 % and 86.7 % by weight, respectively. | | | | |

The disintegration test of these fillings was done as described in Example 1.

| | T2 | T3 | T4 | T5 |
|---|---|---|---|---|
| DISINTEGRATION TEST (time in minutes) | | | | |
| Stage 1: The filling is as one particle and there is no change in its appearance | From 0 min to the time of Stage 2 | From 0 min to the time of Stage 2 | From 0 min to the time of Stage 2 | From 0 min to the time of Stage 2 |
| Stage 2: Appearance of the filling has turned transparent, but the filling is still as one particle | 4.5 min | 5 min | 4 min | 1 min |
| Stage 3: Several (>2) disintegrated particles in the fluid | 12 min | 8 min | 5 min | 2 min |
| Stage 4: The fluid is cloudy | 12 min | 9 min | 8 min | 2 min |

All the capsule fillings, T2-T5, disintegrated during the 20 min follow-up period in the simulated human gastric fluid test. The capsule T3-T5 disintegrated in less than 10 minutes, i.e. in the most preferred disintegration time.

With the fillings according to the invention thus both a high plant stanol ester content and a fast disintegration can be achieved.

Further preferred embodiments are presented herein:
1. A capsule comprising a shell and a filling, wherein the filling comprises plant sterol ester and/or plant stanol ester in an amount of at least 80 % by weight and citrus oil in an amount of at least 2.0 % by weight.
2. The capsule according to embodiment 1, wherein the amount of plant sterol ester and/or plant stanol ester is at least 82 %, preferably at least 85 %, more preferably at least 87 % and most preferably at least 90 % by weight of the filling.
3. The capsule according to embodiment 1 or 2, wherein the amount of plant sterol ester and/or plant stanol ester is at most 98 %, preferably at most 97 %, more preferably at most 96 %, still more preferably at most 95 % and most preferably at most 94 % by weight of the filling.
4. The capsule according to any one of embodiments 1 to 3, wherein the amount of citrus oil is at least 3.0 %, preferably at least 4.0 %, more preferably at least 5.0 % and most preferably at least 6.0 % by weight of the filling.
5. The capsule according to any one of embodiments 1 to 4, wherein the amount of citrus oil is at most 20 %, preferably at most 18 %, more preferably at most 15%, still more preferably at most 13 % and most preferably at most 10 % by weight of the filling.
6. The capsule according to any one of embodiments 1 to 5, wherein the citrus oil is selected from the group consisting of orange oil, grapefruit oil, mandarin oil, lemon oil, lime oil, pomelo oil, bitter orange oil and mixtures of any thereof, preferably of orange oil, grapefruit oil, mandarin oil and mixtures of any thereof, and most preferably of orange oil.
7. The capsule according to any one of embodiments 1 to 6, wherein the citrus oil contains at least 70 %, preferably at least 80 % and most preferably at least 90 % by weight D-limonene
8. The capsule according to any one of embodiments 1 to 7, wherein the weight ratio of plant sterol ester and/or plant stanol ester to citrus oil is at most 45:1, preferably at most 30:1, more preferably at most 25:1 and most preferably at most 18:1.
9. The capsule according to any one of embodiments 1 to 8, wherein the weight ratio of plant sterol ester and/or plant stanol ester to citrus oil is at least 5:1, preferably at least 7:1, more preferably at least 9:1 and most preferably at least 11:1.
10. The capsule according to any one of embodiments 1 to 9, wherein the filling further comprises emulsifier in an amount of 0-15 %, preferably 0.1-15 %, more preferably 1.0-13 %, still more preferably 2.0-10 % and most preferably 3.0-8.0 %, by weight.
11. The capsule according to embodiment 10, wherein the emulsifier is selected from the group consisting of monoglycerides; diglycerides; monoglyceride esters such as acetic, lactic, succinic, citric or diacetyl tartaric acid esters of monoglycerides; lecithins; modified lecithins; polyglycerol esters; polysorbates, sorbitan esters; propylene glycol esters; sugar esters; and mixtures of any thereof, preferably of monoglycerides; diglycerides; lactic, citric or diacetyl tartaric acid esters of monoglycerides; lecithins; modified lecithins; polysorbates, polyglycerol esters; and mixtures of any thereof, more preferably of monoglycerides and/or diglycerides and most preferably of monoglycerides.
12 The capsule according to any one of embodiments 1 to 11, wherein the filling further comprises free plant sterols and/or free plant stanols in an amount of 0-10 %, preferably 0.05-10 %, more preferably 0.05-5.0 %, and most preferably 0.05-3.0 %, by weight.
13 The capsule according to embodiment 12, wherein the amount of free plant sterols and/or free plant stanols is at least 0.5 %, more preferably at least 1.0 % and most preferably at least 2.0 % by weight of the filling.
14 The capsule according to any one of embodiments 1 to 13, wherein the filling further comprises edible fat in an amount of 0-8.0 %, preferably 0.1-8.0 %, more preferably 0.1-4.0 % and most preferably 0.1-2.0 % by weight.
15 The capsule according to any one of embodiments 1 to 14, wherein the capsule is a softgel capsule.
16. The capsule according to any one of embodiments 1 to 15, wherein plant sterol ester and/or plant stanol ester has a solid fat content (SFC) of at least 20 %, preferably at least 25 %, more preferably at least 30 % and most preferably at least 35 % at 20 °C.
17 The capsule according to any one of embodiments 1 to 16, wherein the plant sterol ester and/or plant stanol ester comprises plant stanol ester in an amount of at least 30 %, preferably at least 50 %, more preferably at least 70 % and most preferably at least 90 % by weight.
18. A method for preparing a capsule of any one of embodiments 1 to 17, which comprises at least the following steps:
   a) heating plant sterol ester and/or plant stanol ester, and optionally emulsifier, free plant sterols and/or free plant stanols, edible fat and/or optional other ingredients, preferably to a temperature of at least 40 °C, more preferably to 40-80 °C, to obtain a clear solution, and keeping the solution at this temperature for at least 10 minutes,
   b) mixing citrus oil and plant sterol ester and/or plant stanol ester, and optionally emulsifier, free plant sterols and/or free plant stanols, edible fat and/or optional other ingredients,
   c) keeping the temperature of the mixture such that the mixture remains clear, and using the clear mixture to fill a capsule, preferably a softgel capsule.
19 The capsule according to any one of embodiments 1 to 17 for use as a medicament or for use in lowering serum LDL level or for use in the treatment of cardiovascular disease.

## Claims

1. A capsule comprising a shell and a filling, wherein the filling comprises plant sterol ester and/or plant stanol ester in an amount of at least 80 % by weight and citrus oil in an amount of at least 2.0 % by weight.

2. The capsule according to claim 1, wherein the amount of plant sterol ester and/or plant stanol ester is at least 82 %, preferably at least 85 %, more preferably at least 87 % and most preferably at least 90 % by weight of the filling.

3. The capsule according to claim 1 or 2, wherein the amount of plant sterol ester and/or plant stanol ester is at most 98 %, preferably at most 97 %, more preferably at most 96 %, still more preferably at most 95 % and most preferably at most 94 % by weight of the filling.

4. The capsule according to any one of claims 1 to 3, wherein the amount of citrus oil is at least 3.0 %, preferably at least 4.0 %, more preferably at least 5.0 % and most preferably at least 6.0 % by weight of the filling.

5. The capsule according to any one of claims 1 to 4, wherein the amount of citrus oil is at most 20 %, preferably at most 18 %, more preferably at most 15%, still more preferably at most 13 % and most preferably at most 10 % by weight of the filling.

6. The capsule according to any one of claims 1 to 5, wherein the citrus oil is selected from the group consisting of orange oil, grapefruit oil, mandarin oil, lemon oil, lime oil, pomelo oil, bitter orange oil and mixtures of any thereof, preferably of orange oil, grapefruit oil, mandarin oil and mixtures of any thereof, and most preferably of orange oil.

7. The capsule according to any one of claims 1 to 6, wherein the citrus oil contains at least 70 %, preferably at least 80 % and most preferably at least 90 % by weight D-limonene

8. The capsule according to any one of claims 1 to 7, wherein the weight ratio of plant sterol ester and/or plant stanol ester to citrus oil is at most 45:1, preferably at most 30:1, more preferably at most 25:1 and most preferably at most 18:1.

9. The capsule according to any one of claims 1 to 8, wherein the weight ratio of plant sterol ester and/or plant stanol ester to citrus oil is at least 5:1, preferably at least 7:1, more preferably at least 9:1 and most preferably at least 11:1.

10. The capsule according to any one of claims 1 to 9, wherein the filling further comprises emulsifier in an amount of 0-15 %, preferably 0.1-15 %, more preferably 1.0-13 %, still more preferably 2.0-10 % and most preferably 3.0-8.0 %, by weight.

11. The capsule according to claim 10, wherein the emulsifier is selected from the group consisting of monoglycerides; diglycerides; monoglyceride esters such as acetic, lactic, succinic, citric or diacetyl tartaric acid esters of monoglycerides; lecithins; modified lecithins; polyglycerol esters; polysorbates, sorbitan esters; propylene glycol esters; sugar esters; and mixtures of any thereof, preferably of monoglycerides; diglycerides; lactic, citric or diacetyl tartaric acid esters of monoglycerides; lecithins; modified lecithins; polysorbates, polyglycerol esters; and mixtures of any thereof, more preferably of monoglycerides and/or diglycerides and most preferably of monoglycerides.

12. The capsule according to any one of claims 1 to 11, wherein the filling further comprises free plant sterols and/or free plant stanols in an amount of 0-10 %, preferably 0.05-10 %, more preferably 0.05-5.0 %, and most preferably 0.05-3.0 %, by weight.

13. The capsule according to claim 12, wherein the amount of free plant sterols and/or free plant stanols is at least 0.5 %, more preferably at least 1.0 % and most preferably at least 2.0 % by weight of the filling.

14. The capsule according to any one of claims 1 to 13, wherein the filling further comprises edible fat in an amount of 0-8.0 %, preferably 0.1-8.0 %, more preferably 0.1-4.0 % and most preferably 0.1-2.0 % by weight.

15. The capsule according to any one of claims 1 to 14, wherein the capsule is a softgel capsule.

16. The capsule according to any one of claims 1 to 15, wherein the plant sterol ester and/or plant stanol ester has a solid fat content (SFC) of at least 20 %, preferably at least 25 %, more preferably at least 30 % and most preferably at least 35 % at 20 °C.

17. The capsule according to any one of claims 1 to 16, wherein the plant sterol ester and/or plant stanol ester comprises plant stanol ester in an amount of at least 30 %, preferably at least 50 %, more preferably at least 70 % and most preferably at least 90 % by weight.

18. A method for preparing a capsule of any one of claims 1 to 17, which comprises at least the following steps:
a) heating plant sterol ester and/or plant stanol ester, and optionally emulsifier, free plant sterols and/or free plant stanols, edible fat and/or optional other ingredients, preferably to a temperature of at least 40 °C, more preferably to 40-80 °C, to obtain a clear solution, and keeping the solution at this temperature for at least 10 minutes,
b) mixing citrus oil and plant sterol ester and/or plant stanol ester, and optionally emulsifier, free plant sterols and/or free plant stanols, edible fat and/or optional other ingredients,
c) keeping the temperature of the mixture such that the mixture remains clear, and using the clear mixture to fill a capsule, preferably a softgel capsule.

19. The capsule according to any one of claims 1 to 17 for use as a medicament or for use in lowering serum LDL level or for use in the treatment of cardiovascular disease.

## Patentansprüche

1. Kapsel, umfassend eine Hülle und eine Füllung, wobei die Füllung Pflanzensterolester und/oder Pflanzenstanolester in einer Menge von mindestens 80 Gew.-% und Zitrusöl in einer Menge von mindestens 2,0 Gew.-% umfasst.

2. Kapsel nach Anspruch 1, wobei die Menge an Pflanzensterolester und/oder Pflanzenstanolester mindestens 82 Gew.-%, vorzugsweise mindestens 85 Gew.-%, bevorzugter mindestens 87 Gew.-% und am meisten bevorzugt mindestens 90 Gew.-% der Füllung beträgt.

3. Kapsel nach Anspruch 1 oder 2, wobei die Menge an Pflanzensterolester und/oder Pflanzenstanolester höchstens 98 Gew.-%, vorzugsweise höchstens 97 Gew.-%, stärker bevorzugt höchstens 96 Gew.-%, noch bevorzugter höchstens 95 Gew.-% und am meisten bevorzugt höchstens 94 Gew.-% der Füllung beträgt.

4. Kapsel nach einem beliebigen der Ansprüche 1 bis 3, wobei die Menge an Zitrusöl mindestens 3,0 Gew.-%, vorzugsweise mindestens 4,0 Gew.-%, stärker bevorzugt mindestens 5,0 Gew.-% und am meisten bevorzugt mindestens 6,0 Gew.-% der Füllung beträgt.

5. Kapsel nach einem beliebigen der Ansprüche 1 bis 4, wobei die Menge an Zitrusöl höchstens 20 Gew.-%, vorzugsweise höchstens 18 Gew.-%, stärker bevorzugt höchstens 15 Gew.-%, noch bevorzugter höchstens 13 Gew.-% und am meisten bevorzugt höchstens 10 Gew.-% der Füllung beträgt.

6. Kapsel nach einem beliebigen der Ansprüche 1 bis 5, wobei das Zitrusöl aus der Gruppe bestehend aus Orangenöl, Grapefruitöl, Mandarinenöl, Zitronenöl, Limettenöl, Pomeloöl, Bitterorangenöl und Mischungen aus beliebigen von ihnen, vorzugsweise aus Orangenöl, Grapefruitöl, Mandarinenöl und Mischungen aus beliebigen von ihnen und am meisten bevorzugt aus Orangenöl ausgewählt ist.

7. Kapsel nach einem beliebigen der Ansprüche 1 bis 6, wobei das Zitrusöl mindestens 70 Gew.-%, vorzugsweise mindestens 80 Gew.-% und am meisten bevorzugt mindestens 90 Gew.-% D-Limonen enthält.

8. Kapsel nach einem beliebigen der Ansprüche 1 bis 7, wobei das Gewichtsverhältnis von Pflanzensterolester und/oder Pflanzenstanolester zu Zitrusöl höchstens 45:1, vorzugsweise höchstens 30:1, besonders bevorzugt höchstens 25:1 und am meisten bevorzugt höchstens 18:1 beträgt.

9. Kapsel nach einem beliebigen der Ansprüche 1 bis 8, wobei das Gewichtsverhältnis von Pflanzensterolester und/oder Pflanzenstanolester zu Zitrusöl mindestens 5:1, vorzugsweise mindestens 7:1, bevorzugter mindestens 9:1 und am meisten bevorzugt mindestens 11:1 beträgt.

10. Kapsel nach einem beliebigen der Ansprüche 1 bis 9, wobei die Füllung weiterhin einen Emulgator in einer Menge von 0-15 Gew.-%, vorzugsweise 0,1-15 Gew.-%, bevorzugter 1,0-13 Gew.-%, noch bevorzugter 2,0-10 Gew.-% und am meisten bevorzugt 3,0-8,0 Gew.-% umfasst.

11. Kapsel nach Anspruch 10, wobei der Emulgator aus der Gruppe bestehend aus Monoglyceriden; Diglyceriden; Monoglyceridester wie Essig-, Milch-, Bernstein-, Zitronen- oder Diacetylweinsäureester von Monoglyceriden; Lecithinen; modifizierten Lecithinen; Polyglycerinestern; Polysorbaten, Sorbitanestern; Propylenglykolestern; Zuckerestern; und Mischungen aus beliebigen von ihnen, vorzugsweise aus Monoglyceriden; Diglyceriden; Milch-, Zitronen- oder Diacetylweinsäureestern von Monoglyceriden; Lecithinen; modifizierten Lecithinen; Polysorbaten, Polyglycerinestern; und Mischungen aus beliebigen von ihnen, bevorzugter aus Monoglyceriden und/oder Diglyceriden und am meisten bevorzugt aus Monoglyceriden ausgewählt ist.

12. Kapsel nach einem beliebigen der Ansprüche 1 bis 11, wobei die Füllung ferner freie Pflanzensterole und/oder freie Pflanzenstanole in einer Menge von 0-10 Gew.-%, vorzugsweise 0,05-10 Gew.-%, bevorzugter 0,05-5,0 Gew.-% und am meisten bevorzugt 0,05-3,0 Gew.-% umfasst.

13. Kapsel nach Anspruch 12, wobei die Menge an freien Pflanzensterolen und/oder freien Pflanzenstanolen mindestens 0,5 Gew.-%, bevorzugter mindestens 1,0 Gew.-% und am meisten bevorzugt mindestens 2,0 Gew.-% der Füllung beträgt.

14. Kapsel nach einem beliebigen der Ansprüche 1 bis 13, wobei die Füllung ferner Speisefett in einer Menge von 0-8,0 Gew.-%, vorzugsweise 0,1-8,0 Gew.-%, bevorzugter 0,1-4,0 Gew.-% und am meisten bevorzugt 0,1-2,0 Gew.-% umfasst.

15. Kapsel nach einem beliebigen der Ansprüche 1 bis 14, wobei die Kapsel eine Weichkapsel ist.

16. Kapsel nach einem beliebigen der Ansprüche 1 bis 15, wobei der Pflanzensterolester und/oder der Pflanzenstanolester einen Feststofffettgehalt (SFC) von mindestens 20 %, vorzugsweise mindestens 25 %, besonders bevorzugt mindestens 30 % und am meisten bevorzugt mindestens 35 % bei 20 °C aufweist.

17. Kapsel nach einem beliebigen der Ansprüche 1 bis 16, wobei der Pflanzensterolester und/oder der Pflanzenstanolester Pflanzenstanolester in einer Menge von mindestens 30 Gew.-%, vorzugsweise mindestens 50 Gew.-%, bevorzugter mindestens 70 Gew.-% und am meisten bevorzugt mindestens 90 Gew.-% umfasst.

18. Verfahren zum Herstellen einer Kapsel nach einem beliebigen der Ansprüche 1 bis 17, das mindestens die folgenden Schritte umfasst:
a) Erwärmen von Pflanzensterolester und/oder Pflanzenstanolester und gegebenenfalls Emulgator, freien Pflanzensterolen und/oder freien Pflanzenstanolen, Speisefett und/oder gegebenenfalls anderen Bestandteilen, vorzugsweise auf eine Temperatur von mindestens 40 °C, besonders bevorzugt auf 40-80 °C, um eine klare Lösung zu erhalten, und Halten der Lösung bei dieser Temperatur für mindestens 10 Minuten,
b) Mischen von Zitrusöl und Pflanzensterolester und/oder Pflanzenstanolester und gegebenenfalls Emulgator, freien Pflanzensterolen und/oder freien Pflanzenstanolen, Speisefett und/oder gegebenenfalls anderen Bestandteilen,
c) Halten der Temperatur der Mischung, so dass die Mischung klar bleibt und Verwenden der klaren Mischung zum Befüllen einer Kapsel, vorzugsweise einer Weichkapsel.

19. Kapsel nach einem beliebigen der Ansprüche 1 bis 17 zur Verwendung als Medikament oder zur Verwendung bei der Senkung des LDL-Spiegels im Serum oder zur Verwendung bei der Behandlung von Herz-Kreislauf-Erkrankungen.

## Revendications

1. Capsule comprenant une coque et un remplissage, dans laquelle le remplissage comprend un ester de stérol végétal et/ou un ester de stanol végétal en une quantité d'au moins 80 % en poids et de l'huile d'agrume en une quantité d'au moins 2,0 % en poids.

2. Capsule selon la revendication 1, dans laquelle la quantité d'ester de stérol végétal et/ou d'ester de stanol végétal est d'au moins 82 %, de préférence d'au moins 85 %, plus préférablement d'au moins 87 % et le plus préférablement d'au moins 90 % en poids par rapport au remplissage.

3. Capsule selon la revendication 1 ou 2, dans laquelle la quantité d'ester de stérol végétal et/ou d'ester de stanol végétal est d'au plus 98 %, de préférence d'au plus 97 %, plus préférablement d'au plus 96 %, encore plus préférablement d'au plus 95 % et le plus préférablement d'au plus 94 % en poids par rapport au remplissage.

4. Capsule selon l'une quelconque des revendications 1 à 3, dans laquelle la quantité d'huile d'agrumes est d'au moins 3,0 %, de préférence d'au moins 4,0 %, plus préférablement d'au moins 5,0 % et le plus préférablement d'au moins 6,0 % en poids par rapport au remplissage.

5. Capsule selon l'une quelconque des revendications 1 à 4, dans laquelle la quantité d'huile d'agrumes est d'au plus 20 %, de préférence d'au plus 18 %, plus préférablement d'au plus 15 %, encore plus préférablement d'au plus 13 % et le plus préférablement d'au plus 10 % en poids par rapport au remplissage.

6. Capsule selon l'une quelconque des revendications 1 à 5, dans laquelle l'huile d'agrume est choisie dans le groupe constitué par l'huile d'orange, l'huile de pamplemousse, l'huile de mandarine, l'huile de citron, l'huile de limette, l'huile de pomelo, l'huile d'orange amère et les mélanges de plusieurs quelconques d'entre eux, de préférence l'huile d'orange, l'huile de pamplemousse, l'huile de mandarine et les mélanges de plusieurs quelconques d'entre eux, et le plus préférablement l'huile d'orange.

7. Capsule selon l'une quelconque des revendications 1 à 6, dans laquelle l'huile d'agrumes contient au moins 70 %, de préférence au moins 80 % et le plus préférablement au moins 90 % en poids de D-limonène.

8. Capsule selon l'une quelconque des revendications 1 à 7, dans laquelle le rapport en poids de l'ester de stérol végétal et/ou de l'ester de stanol végétal à l'huile d'agrume est d'au plus 45:1, de préférence d'au plus 30:1, plus préférablement d'au plus 25:1, et le plus préférablement d'au plus 18:1.

9. Capsule selon l'une quelconque des revendications 1 à 8, dans laquelle le rapport en poids de l'ester de stérol végétal et/ou de l'ester de stanol végétal à l'huile d'agrume est d'au moins 5:1, de préférence d'au moins 7:1, plus préférablement d'au moins 9:1, et le plus préférablement d'au moins 11:1.

10. Capsule selon l'une quelconque des revendications 1 à 9, dans laquelle le remplissage comprend en outre un émulsifiant en une quantité de 0 à 15 %, de préférence de 0,1 à 15 %, plus préférablement de 1,0 à 13 %, encore plus préférablement de 2,0 à 10 % et le plus préférablement 3,0 à 8,0 % en poids.

11. Capsule selon la revendication 10, dans laquelle l'émulsifiant est choisi dans le groupe constitué par les monoglycérides ; les diglycérides ; les esters de monoglycérides tels que les esters d'acide acétique, lactique, succinique, citrique ou diacétyltartrique de monoglycérides ; les lécithines ; les lécithines modifiées ; les esters de polyglycérol ; les polysorbates, les esters de sorbitan ; les esters de propylèneglycol ; les esters de sucre ; et les mélanges de plusieurs quelconques d'entre eux, de préférence les monoglycérides ; les diglycérides ; les esters d'acide lactique, citrique ou diacétyltartrique de monoglycérides ; les lécithines ; les lécithines modifiées ; les polysorbates ; les esters de polyglycérol ; et les mélanges de plusieurs quelconques d'entre eux, plus préférablement les monoglycérides et/ou les diglycérides et le plus préférablement les monoglycérides.

12. Capsule selon l'une quelconque des revendications 1 à 11, dans laquelle le remplissage comprend en outre des stérols végétaux libres et/ou des stanols végétaux libres en une quantité de 0 à 10 %, de préférence de 0,05 à 10 %, plus préférablement de 0,05 à 5,0 %, et le plus préférablement de 0,05 à 3,0 % en poids.

13. Capsule selon la revendication 12, dans laquelle la quantité de stérols végétaux libres et/ou de stanols végétaux libres est d'au moins 0,5 %, plus préférentiellement d'au moins 1,0 % et le plus préférablement d'au moins 2,0 % en poids par rapport au remplissage.

14. Capsule selon l'une quelconque des revendications 1 à 13, dans laquelle le remplissage comprend en outre une matière grasse alimentaire en une quantité de 0 à 8,0 %, de préférence de 0,1 à 8,0 %, plus préférablement de 0,1 à 4,0 % et le plus préférablement de 0,1 à 2,0 % en poids.

15. Capsule selon l'une quelconque des revendications 1 à 14, dans laquelle la capsule est une capsule à enveloppe molle.

16. Capsule selon l'une quelconque des revendications 1 à 15, dans laquelle l'ester de stérol végétal et/ou l'ester de stanol végétal a une teneur en corps gras solides (SFC) d'au moins 20 %, de préférence d'au moins 25 %, plus préférablement d'au moins 30 % et le plus préférablement d'au moins 35 % à 20 °C.

17. Capsule selon l'une quelconque des revendications 1 à 16, dans laquelle l'ester de stérol végétal et/ou l'ester de stanol végétal comprend un ester de stanol végétal en une quantité d'au moins 30 %, de préférence d'au moins 50 %, plus préférablement d'au moins 70 % et le plus préférablement d'au moins 90 % en poids.

18. Procédé de préparation d'une capsule selon l'une quelconque des revendications 1 à 17, qui comprend au moins les étapes suivantes, consistant à :
a) chauffer l'ester de stérol végétal et/ou l'ester de stanol végétal, et éventuellement l'émulsifiant, les stérols végétaux libres et/ou les stanols végétaux libres, la matière grasse alimentaire et/ou d'autres ingrédients éventuels, de préférence à une température d'au moins 40 °C, plus préférablement de 40 à 80 °C, pour obtenir une solution limpide, et maintenir la solution à cette température pendant au moins 10 minutes,
b) mélanger l'huile d'agrume et l'ester de stérol végétal et/ou l'ester de stanol végétal, et éventuellement l'émulsifiant, les stérols végétaux libres et/ou les stanols végétaux libres, la matière grasse alimentaire et/ou les autres ingrédients éventuels,
c) maintenir la température du mélange de telle sorte que le mélange reste limpide et utiliser le mélange limpide pour remplir une capsule, de préférence une capsule à enveloppe molle.

19. Capsule selon l'une quelconque des revendications 1 à 17 pour une utilisation comme médicament ou pour une utilisation pour réduire le taux de LDL sérique ou pour une utilisation pour le traitement d'une maladie cardiovasculaire.
